(19) Europäisches Patentamt
European Patent Office
Office européen des brevets

(11) Veröffentlichungsnummer: **0 090 277**
**B1**

(12) # EUROPÄISCHE PATENTSCHRIFT

(45) Veröffentlichungstag der Patentschrift: **24.10.84**

(51) Int. Cl.³: **C 07 C 13/28,** C 07 C 1/24

(21) Anmeldenummer: **83102650.5**

(22) Anmeldetag: **17.03.83**

(54) **Verfahren zur Herstellung von 2,2-Dicyclohexenylpropan.**

(30) Priorität: **26.03.82 DE 3211304**

(43) Veröffentlichungstag der Anmeldung: **05.10.83 Patentblatt 83/40**

(45) Bekanntmachung des Hinweises auf die Patenterteilung: **24.10.84 Patentblatt 84/43**

(84) Benannte Vertragsstaaten: **DE FR GB**

(56) Entgegenhaltungen: **DE - B - 1 099 733**

(73) Patentinhaber: **BAYER AG, Konzernverwaltung RP Patentabteilung, D-5090 Leverkusen 1 Bayerwerk (DE)**

(72) Erfinder: **Rauleder, Gebhard, Dr., Mozartstrasse 20, D-5657 Haan (DE)**
Erfinder: **Waldmann, Helmut, Dr., Henry-T.-von Boettinger Strasse 15, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 090 277 B1

ACTORUM AG

**Beschreibung**

Die vorliegende Erfindung betrifft ein Verfahren zur Herstellung von 2,2-Dicyclohexenylpropan durch Dehydratisieren von perhydriertem 4,4'-Dioxydiphenylpropan (im folgenden wird 4,4'-Dioxydiphenylpropan auch als Bisphenol A und perhydriertes 4,4'-Dioxydiphenylpropan auch als 4,4'-Dioxydicyclohexylpropan bezeichnet).

2,2-Dicyclohexenylpropan ist eine bekannte chemische Verbindung, die bei der Herstellung von Polymeren als organisches Zwischenprodukt und zur Herstellung von 2,2-Dicyclohexenylpropandiepoxid Verwendung findet.

Zur Herstellung von 2,2-Dicyclohexenylpropan durch Wasserabspaltung aus perhydriertem Bisphenol A gemäss folgender Gleichung

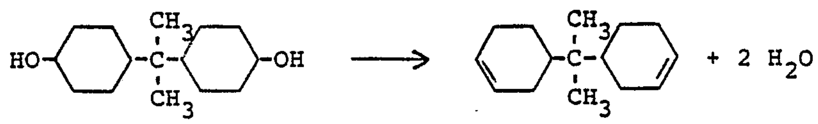

sind bereits Vorschläge gemacht worden. So wird in Beispiel 1 der DE-PS Nr. 857961 ein derartiges Verfahren beschrieben, bei dem perhydriertes Bisphenol A mit annähernd der doppelten Gewichtsmenge an wasserfreiem Kaliumbisulfat 8 h lang auf 160° C erhitzt wird. Anschliessend daran ist das Gemisch mit Wasser zu waschen und das organische Produkt zu destillieren. Ausbeuten sind keine angegeben. Für eine technische Anwendung ist dieses Verfahren jedoch wenig geeignet, da nach der Aufarbeitung grosse Mengen an umweltbelastenden wässerigen Salzlösungen anfallen.

Auch das in der DE-AS Nr. 1099733 beschriebene Verfahren erbringt keinen Vorteil. Im Beispiel A wird dort die Herstellung des 2,2-Dicyclohexenylpropans durch Dehydratisieren von 340 g perhydriertem Bisphenol A mit 4 g β-Naphthalinsulfonsäure und 900 g eines gemahlenen Dehydratisierungsgemisches beschrieben, das zu gleichen Teilen aus Kaliumbisulfat und trockenem Sand besteht. Das gebildete Wasser wird während der Reaktion ständig abdestilliert. Nach Reaktionsende wird mit Ether aufgenommen, mit Wasser neutral gewaschen, mit Calciumchlorid getrocknet und der Ether im Vakuum abdestilliert. Die Nachteile dieses Verfahrens sind die gleichen wie die oben erwähnten, die Ausbeute beträgt etwa 60% der Theorie.

Es wurde nun ein Verfahren zur Herstellung von 2,2-Dicyclohexenylpropan aus 4,4'-Dioxydicyclohexylpropan gefunden, das dadurch gekennzeichnet ist, dass man 4,4'-Dioxydicyclohexylpropan in Gegenwart einer starken Säure erhitzt, bei einem Druck von 1 bis 50 mbar und Temperaturen im Bereich von 120 bis 220° C das entstehende 2,2-Dicyclohexenylpropan zusammen mit dem gebildeten Wasser kontinuierlich aus dem Reaktionsgemisch abdestilliert und aus dem Kopfprodukt dieser Destillation das Wasser abtrennt.

Das Ausgangsprodukt für das erfindungsgemässe Verfahren, das perhydrierte 4,4'-Dioxydiphenylpropan, ist beispielsweise durch Hydrierung von 4,4'-Dioxydiphenylpropan (= Bisphenol A) erhältlich (s. DE-AS Nr. 1099733).

Als starke Säuren kommen für das erfindungsgemässe Verfahren alle starken Säuren in Frage, die bei den Reaktionsbedingungen stabil sind. Vorzugsweise werden schwerflüchtige starke Säuren eingesetzt, beispielsweise Arylsulfonsäuren oder Mineralsäuren, wie Schwefelsäure oder Phosphorsäure. Vorzugsweise wird p-Toluolsulfonsäure eingesetzt.

Die Konzentration der Säure im Reaktionsgemisch kann in weiten Grenzen schwanken. Geeignet sind z.B. Konzentrationen von 0,5 bis 10 Gew.-%. Bevorzugt sind Konzentrationen von 0,75 bis 5 Gew.-%, besonders bevorzugt solche von 1 bis 2 Gew.-%.

Das erfindungsgemässe Verfahren kann im allgemeinen so durchgeführt werden, dass man perhydriertes Bisphenol A und die starke Säure zusammengibt, dann einen Druck im Bereich von 1 bis 50 mbar einstellt, dann auf Temperaturen im Bereich von 120 bis 220° C erhitzt und das entstehende 2,2-Dicyclohexenylpropan zusammen mit dem entstehenden Wasser abdestilliert. Die Einstellung des Druckes und der Temperatur können auch in umgekehrter Reihenfolge oder gleichzeitig vorgenommen werden. Innerhalb der angegebenen Bereiche sind Druck und Temperatur in jedem Fall so zu kombinieren, dass 2,2-Dicyclohexenylpropan und Wasser abdestillieren. Vorzugsweise arbeitet man bei Temperaturen von 160 bis 210°C und bei Drucken von 5 bis 50 mbar, besonders bevorzugt bei Temperaturen von 185 bis 200°C und Drucken von 10 bis 20 mbar.

Neben der Arbeitsweise unter isothermen Bedingungen, d.h. Einhaltung einer einheitlichen Temperatur während der Reaktion, kann man die Umsetzung auch unter Ausbildung eines sog. Temperaturgradienten durchführen, d.h. bei sich innerhalb der angegebenen Grenzen ändernden Temperaturen.

Das abdestillierende 2,2-Dicyclohexenylpropan und das abdestillierende Wasser können gemeinsam oder getrennt kondensiert werden. Bei gemeinsamer Kondensation bilden sich zwei Phasen aus, und nach Abtrennung der wässerigen Phase hinterbleibt 2,2-Dicyclohexenylpropan. Bei getrennter Kondensation scheidet sich zunächst bei höherer Temperatur nur 2,2-Dicyclohexenylpropan ab und anschliessend bei tieferer Temperatur das Wasser. Falls flüchtige starke Säuren eingesetzt wurden, befinden sich diese nach der Reaktion im allgemeinen in der Wasserphase und können zusammen mit dieser entfernt werden. Wenn die bevorzugten schwerflüchtigen starken Säuren eingesetzt werden, so verbleiben diese im Sumpf der Destillation und können für neue Ansätze wieder verwendet werden.

In einer bevorzugten technischen Ausführungsform des erfindungsgemässen Verfahrens wird perhydriertes Bisphenol A zusammen mit etwa 1 bis 2 Gew.-% p-Toluolsulfonsäure vorgelegt und unter einem Druck von 10 bis 20 mbar auf Temperaturen von 190 bis 200°C erhitzt. Das durch die ablaufende Reaktion gebildete 2,2-Dicyclohexenylpropan wird zusammen mit dem gebildeten Wasser kontinuierlich aus dem Reaktionsgemisch abdestilliert, während in den Reaktor ständig frisches perhydriertes Bisphenol A zudosiert wird. Das dabei am Kopf der Destillation anfallende Produkt, das im wesentlichen nur aus 2,2-Dicyclohexenylpropan und Wasser besteht, wird abgekühlt und einem Abscheider zugeführt, in dem sich zwei Phasen ausbilden. Die wässerige Phase wird abgetrennt, die verbleibende organische Phase ist reines oder nahezu reines 2,2-Dicyclohexenylpropan.

In einer anderen bevorzugten technischen Ausführungsform erfolgt die Kondensation so, dass in einem ersten Kondensator nur das 2,2-Dicyclohexenylpropan kondensiert wird, während das Wasser bei tieferer Temperatur in einem zweiten Kondensator erhalten wird.

Mit dem erfindungsgemässen Verfahren kann man 2,2-Dicyclohexenylpropan in einer Ausbeute von über 95% der Theorie, mit einem Wassergehalt von unter 0,1 Gew.-% und einer Reinheit von über 99% erhalten. Das abgetrennte Reaktionswasser kann gegebenenfalls durch einfaches Anstrippen von geringen Mengen in ihm vorhandenen organischen Verbindungen befreit werden. Der Vorteil des erfindungsgemässen Verfahrens besteht darin, dass 2,2-Dicyclohexenylpropan in einfacher Weise mit hohen Ausbeuten und hoher Reinheit wirtschaftlich zugänglich ist und die Verwendung von grossen Salzmengen, die zu umweltbelastenden Abwässern führen, vermieden werden können.

*Beispiel:*

In einem Rührreaktor mit aufgesetzter Destillationskolonne wurden 600 g perhydriertes Bisphenol A und 9 g p-Toluolsulfonsäure vorgelegt. Unter Rühren wurde ein Druck von 15 mbar eingestellt und das Gemisch auf 195°C erhitzt. Das entstehende 2,2-Dicyclohexenylpropan destillierte zusammen mit dem Reaktionswasser ab. Bei Beginn der Destillation wurde die Zudosierung von frischem perhydriertem Bisphenol A in Betrieb genommen. Die Pumpe wurde so eingestellt, dass sich im Reaktor ein konstanter Stand an Reaktionsgemisch einstellte. Es wurden stündlich 175 g perhydriertes Bisphenol A zudosiert. Am Kopf der Kolonne erhielt man in einem ersten Kondensator 2,2-Dicyclohexenylpropan und in einem zweiten Kondensator das abgetrennte Reaktionswasser, das in einem Abscheider von kleinen Mengen mitgerissenem 2,2-Dicyclohexenylpropan befreit wurde. Letzteres wurde in die Destillationskolonne zurückgeführt.

2,2-Dicyclohexenylpropan wurde stündlich in einer Menge von 143,8 g erhalten, das entspricht einer Ausbeute von 96,3% der Theorie. Die Reinheit des abgetrennten 2,2-Dicyclohexenylpropans war 99,6%.

## Patentansprüche

1. Verfahren zur Herstellung von 2,2-Dicyclohexenylpropan aus 4,4'-Dioxydicyclohexylpropan, dadurch gekennzeichnet, dass man 4,4'-Dioxydicyclohexylpropan in Gegenwart einer starken Säure erhitzt, bei einem Druck von 1 bis 50 mbar und Temperaturen im Bereich von 120 bis 220°C das entstehende 2,2-Dicyclohexenylpropan zusammen mit dem gebildeten Wasser kontinuierlich aus dem Reaktionsgemisch abdestilliert und aus dem Kopfprodukt dieser Destillation das Wasser abtrennt.

2. Verfahren nach Anspruch 1, dadurch gekennzeichnet, dass man, bezogen auf das Reaktionsgemisch, 0,5 bis 10 Gew.-% starke Säure einsetzt.

3. Verfahren nach den Ansprüchen 1 und 2, dadurch gekennzeichnet, dass man als starke Säure p-Toluolsulfonsäure, Schwefelsäure oder Phosphorsäure einsetzt.

4. Verfahren nach den Ansprüchen 1 bis 3, dadurch gekennzeichnet, dass man bei Temperaturen von 160 bis 210°C und bei Drucken von 5 bis 50 mbar arbeitet.

5. Verfahren nach den Ansprüchen 1 bis 4, dadurch gekennzeichnet, dass man das abdestillierende 2,2-Dicyclohexenylpropan und das abdestillierende Wasser gemeinsam kondensiert und die dabei anfallende wässerige Phase abtrennt.

6. Verfahren nach den Ansprüchen 1 bis 4, dadurch gekennzeichnet, dass man das abdestillierende 2,2-Dicyclohexenylpropan und das abdestillierende Wasser getrennt kondensiert und in einer ersten Kondensation das 2,2-Dicyclohexenylpropan und in einer zweiten, bei tieferer Temperatur betriebenen Kondensation das Wasser abtrennt.

## Claims

1. Process for the preparation of 2,2-dicyclohexenylpropane from 4,4'-dioxydicyclohexylpropane, characterised in that 4,4'-dioxydicyclohexylpropane is heated in the presence of a strong acid, under a pressure from 1 to 50 mbar and at temperatures in the range from 120 to 220°C, the 2,2-dicyclohexenylpropane produced, together with the water formed, is distilled continuously out of the reaction mixture and the water is separated out of the top product from this distillation.

2. Process according to Claim 1, characterised in that 0.5 to 10% by weight, relative to the reaction mixture, of strong acid are employed.

3. Process according to Claims 1 and 2, characterised in that p-toluenesulphonic acid, sulphuric acid or phosphoric acid is employed as the strong acid.

4. Process according to Claims 1 to 3, characterised in that temperatures from 160 to 210°C and pressures from 5 to 50 mbar are used.

5. Process according to Claims 1 to 4, characterised in that the 2,2-dicyclohexenylpropane distilling off and the water distilling off are

condensed together and the aqueous phase thereby resulting is separated off.

6. Process according to Claims 1 to 4, characterised in that the 2,2-dicyclohexenylpropane distilling off and the water distilling off are condensed separately and the 2,2-dicyclohexenylpropane is separated off in a first condensation and the water is separated off in a second condensation carried out at a lower temperature.

**Revendications**

1. Procédé pour la préparation de 2,2-dicyclohexenylpropane à partir de 4,4'-dioxydicyclohexylpropane, caractérisé en ce qu'on chauffe du 4,4'-dioxydicyclohexylpropane en présence d'un acide fort, élimine en continu par distillation à partir du mélange réactionnel à une pression de 1 à 50 mbar et à des températures comprises entre 120 et 220°C le 2,2-dicyclohexenylpropane résultant en même temps que l'eau formée et sépare l'eau du produit de tête de cette distillation.

2. Procédé selon la revendication 1, caractérisé en ce que, par rapport au mélange réactionnel, on met en œuvre 0,5 à 10% en poids d'acide fort.

3. Procédé selon les revendications 1 et 2, caractérisé en ce qu'on met en œuvre comme acide fort de l'acide p-toluène sulfonique, de l'acide sulfurique ou de l'acide phosphorique.

4. Procédé selon les revendications 1 à 3, caractérisé en ce qu'on opère à des températures de 160 à 210°C et à des pressions de 5 à 50 mbar.

5. Procédé selon les revendications 1 à 4, caractérisé en ce qu'on condense ensemble le 2,2-dicyclohexenylpropane et l'eau en voie de distillation et en ce qu'on sépare la phase aqueuse ainsi produite.

6. Procédé selon les revendications 1 à 4, caractérisé en ce qu'on condense séparément le 2,2-dicyclohexenylpropane et l'eau en voie de distillation et qu'on sépare le 2,2-dicyclohexenylpropane au cours d'une première condensation et l'eau au cours d'une deuxième condensation menée à une température plus basse.